# EUROPEAN PATENT APPLICATION

(11) **EP 3 292 908 A1**
(43) Date of publication of application: **14.03.2018**
(21) Application number: 17173619.2
(22) Date of filing: 31.05.2017
(51) Int. Cl.: B01J 13/14, A61K 9/51, B01J 13/22, A61K 9/50

(54) **BIOCOMPATIBLE CROSS-LINKED MICRO-NANO MATERIAL USED FOR DRUG LOADING AND SUSTAINED RELEASE AND CONSTRUCTION METHOD THEREOF**

(30) Priority: 13.09.2016 CN 201610819800
(71) Applicant: Wenzhou Institute of Biomaterials and Engineering, Wenzhou, Zhejiang 325001 (CN)
(72) Inventor: LIU, Zhe, Wenzhou, Zhejiang 325001 (CN); CHEN, Wan-er, Wenzhou, Zhejiang 325001 (CN)
(74) Representative: TLIP Limited

(57) **Abstract**

The present invention discloses a construction process of biocompatible micro-nano material which can be used for drug loading and sustained release. The constructed biomaterial is a polyacrylate-based microbubble and nanoparticle containing genipin crosslinked chitosan; using the adriamycin as model drug, the drug loading and release properties are investigated. The micro-nanoparticle material constructed by this method has improved the encapsulation efficiency and loading rate of the drug to a certain extent, which can control the in vitro release rate of the drug appropriately.

## Description

### FIELD OF THE INVENTION

The present invention relates to a construction process of biocompatible micro-nano material which can be used for drug loading and sustained release.

### BACKGROUND

The following description of the background art is merely for the purpose of assisting in the understanding of the present invention and is not intended to limit the scope of the present application.

Surface encapsulation is one of the important methods for functional modification of micro-nanoparticles. After micro-nanoparticles are encapsulated with appropriate material, their size, surface chargeability, stability, and expansion coefficient may be changed compared with those before encapsulation. In addition, the encapsulation material can effectively impart specific biochemical properties to micro-nanoparticles, to facilitate the functional modification of micro-nano materials. However, the biocompatibility of the encapsulated material will directly affect the bioactivity of the final micro-nanomaterials. The modification of the encapsulant determines the prospect of the micro-nanomaterials. Therefore, it is prerequisite to find an encapsulant with excellent biocompatibility and high modification.

Similar to surface encapsulation, surface crosslinking is also an important approach to modify micro-nanoparticles. Different from the encapsulation method, the size of micro-nanoparticle becomes usually larger after encapsulation, while it becomes smaller after crosslinking. It is important that the change of material compactness after crosslinking results in changes of a series of physical and chemical activity and biological activity such as structural stability, hydrophilicity, biodegradability, toxicity, etc., which extensively expands their applications in the biomedical field. In addition, the degree of cross-linking of the material can greatly affect the loading efficiency and release behaviors of drugs. Therefore, the surface cross-linking is one of the important means to improve the loading efficiency of micro-nanoparticle and make the drug encapsulated on the carrier to achieve sustained release and controlled release.

Aldehyde amine condensation is a common modification method for amino-based biomaterials, and the most commonly used aldehyde crosslinking agent is glutaraldehyde, but it is a flammable and explosive product and produces harm and threat to environment and human health. In addition, the glutaraldehyde cross-linked biomaterial will generate aldehyde-containing intermediates after in vivo degradation that makes protein denaturation in the cells, thus, the material biocompatibility becomes worse.

### SUMMARY

One object of the present invention is to overcome the shortcomings and defects of the prior art, and provide a universal method of constructing a micro-nanoparticle material, that is, to provide a construction process of biocompatible micro-nano material which can be used for drug loading and sustained release.

Another object of the invention is to compare the change of particle size and potential and other properties before and after encapsulation and crosslinking, and the change of loading efficiency and release behavior before and after crosslinking.

To achieve the first object of the invention, the micro-nanoparticles are further polyacrylate microbubbles and nanoparticles.

Further, the biocompatible material for encapsulation is chitosan.

Further, the biocompatible crosslinking agent used is genipin.

Further, the construction process includes three steps: Chitosan encapsulates the micro-nanoparticles, genipin cross-links the chitosan-encapsulated micro-nanoparticles, and genipin cross-links the drug load on the chitosan-encapsulated micro-nanoparticles.

Further, the process of chitosan encapsulation of micro-nanoparticles includes the following steps:
a. Mix the aqueous solution of micro-nanoparticle with a 5% chitosan solution at the volume ratio of 0.1∼1.0 for 1 to 3 hours to form a chitosan-encapsulated micro-nanoparticle;
b. Centrifuge to separate chitosan-encapsulated micro-nanoparticles, and wash to remove the non-encapsulated chitosan using pure water;
c. after concentration, redisperse the micro-nanoparticle in phosphate buffer(PPS).

Further, the process of genipin crosslinking of chitosan-encapsulated micro-nanoparticles includes the following steps:
a. Mix the chitosan-encapsulated micro-nanoparticle solution with 0.1 mol/L genipin solution at the volume ratio of 0.05∼0.5 for 1 to 24 hours;
b. Centrifuge to separate micro-nanoparticles, and wash to remove the unreacted genipin using pure water;
c. after concentration, redisperse the drug-loaded micro-nanoparticle in phosphate buffer(PPS).

Further, the process of drug loading on chitosan-encapsulated micro-nanoparticles includes the following steps:
a. Mix the chitosan-encapsulated micro-nanoparticle solution with appropriate amount of aqueous solution of a water-soluble drug, then add 0.1 mol/L genipin solution at the volume ratio of 0.05∼0.5 for 1 to 24 hours;
b. Centrifuge to separate micro-nanoparticles, and wash to remove the unreacted genipin and non-encapsulated drug using pure water;
c. after concentration, redisperse the drug-loaded micro-nanoparticles in phosphate buffer(PPS).

The chitosan used for encapsulation of micro-nanoparticle has rich source, which is the second largest renewable resource only inferior to cellulose. It is the sole natural cationic polymer in nature, with good biocompatibility and biodegradability; in addition, it has anticoagulant and antibacterial activity. Chitosan molecules contain a large number of amino and hydroxyl groups. After encapsulated by chitosan, the hydrophilicity of micro-nano-materials will be increased, and their biocompatibility and stability will be enhanced.

Genipin is a product of geniposide hydrolyzed by β-glucosidase, and it is an excellent natural biological cross-linking agent. It can be used to produce biomaterials with proteins, collagens, gelatin and chitosan and other cross-linking agents, such as artificial bones, wound bandage materials, etc.. Its toxicity is much lower than glutaraldehyde and other commonly used chemical cross-linking agents. In addition, genipin itself has antibacterial, anti-inflammatory, antioxidant activity, and can protect neurons and effectively control type II diabetes, etc.. After crosslinked with micro-nanoparticles, genipin can enhance the mechanical strength and tensile strength of biomaterials, and maintain part of its original physiological activities (such as antibacterial activity).

The preparation process is simple, environmentally -friendly, free of contamination of three wastes and radiation and noises, with mild condition and low energy consumption; its separation and purification processes are simple and the resulting system is stable and easy to preserve, therefore, it is a universal process of highly biocompatible cross-linking of micro-nanoparticles.

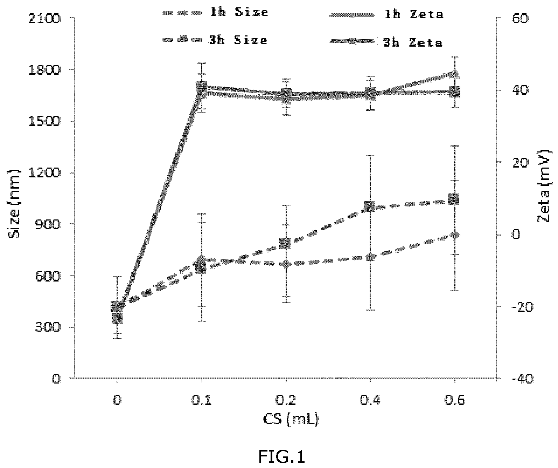

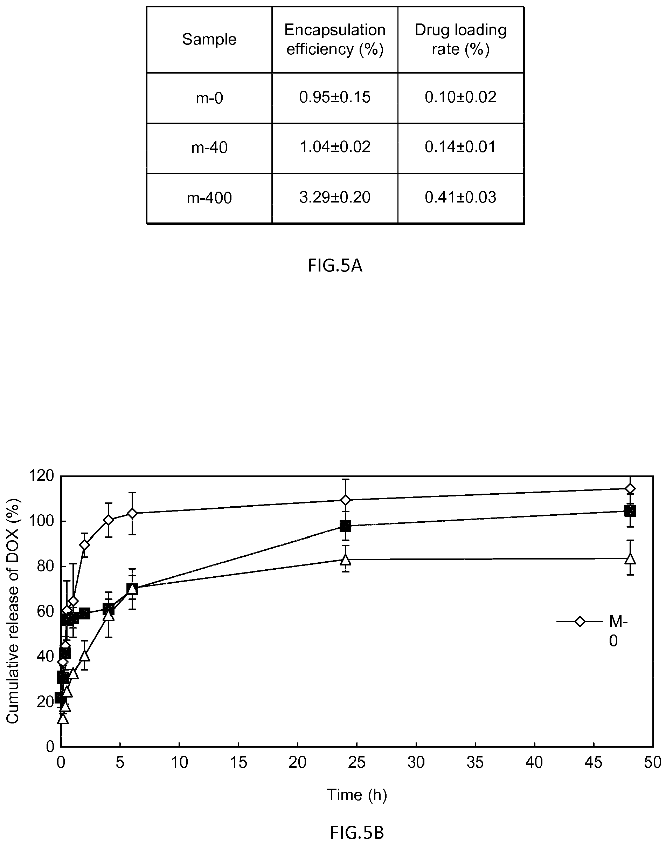

In order to achieve the second object of the invention, the size and potential of micro-nanoparticles before and after crosslinking are detected by a particle size analyzer. Results show that, after encapsulation of chitosan, the particle size of micro-nano material is changed, the potential is obviously increased or even converted from negative charge to positive charge; after genipin crosslinking, the size and potential of micro-nanoparticles are decreased to varying degrees (Fig.1 ∼ 4). Through encapsulating model drug doxorubicin hydrochloride into micro-nanoparticle material by genipin crosslinking, the effect of different cross-linking agents on the loading efficiency of the drug is compared. In the in vitro release test of drugs, the release behaviors of drugs in different cross-linking materials are compared, results show that, the increased amount of cross-linking agents can effectively enhance the loading efficiency of drugs (Fig.5a), and slow down the release of drugs encapsulated in the material with high crosslinking degree (FIG.5b).

The encapsulation and crosslinking of micro-nanoparticles can effectively improve the biocompatibility and stability of micro-nanoparticles, effectively enhance the loading efficiency and adjust the drug release behavior. It is expected to be extensively used in bio-labeling and tracing, medical imaging, integrated diagnosis and treatment, etc., to produce good economic and social benefits in the life health and personalized healthcare, etc..

The present invention is further described in combination with drawings and specific embodiments.

### BRIEF DESCRIPTION OF THE DRAWINGS

FIG.1 Changes in particle size and potential of nanoparticles in 1-3 hours after encapsulation of nanoparticles with chitosan at different volume ratios (Example 1)
FIG.2 Changes in particle size and potential of microbubbles in 3 hours after encapsulation of microbubbles with chitosan at different volume ratios (Example 2)
FIG.3 Changes in the size and potential of the nanoparticles in 1-20 hours after crosslinking of chitosan-encapsulated nanoparticles with genipin at different volume ratios (Example 3)
FIG. 4 Changes in the size and potential of the microbubbles in 1-20 hours after crosslinking of chitosan-encapsulated microbubbles with genipin at different volume ratios (Example 4)
FIG.5a Comparison of encapsulation efficiency and loading efficiency after encapsulation of model drug adriamycin hydrochloride to the microbubbles with cross-linking agents at different doses (Example 5)
FIG.5b The release curve of model drug adriamycin hydrochloride loaded onto microbubbles of different crosslinking degrees in 48 hours (Example 5)

### DETAILED DESCRIPTION

The present invention is described by way of examples, which are used to further explain the invention rather than limit the scope of protection of the invention. Technicians skilled in the art can make some non-essential improvements and adjustments according to the above described contents herein.

All raw materials used in the present invention are commercially available.

### Example 1

Four tubes of 1 mL of polyacrylate nanoparticles (prepared by their own) are taken, added to 0.1, 0.2, 0.4, 0.6 mL of 5wt% chitosan solution respectively, after mixed 1hour and 3 hours, centrifuged to separate the nano-particles, then the non-encapsulated chitosan is washed with pure water (the washing process is known in the industry, usually washed with pure water for three times repeatedly, non-special washing method). The particle size and potential before and after crosslinking are measured by a particle size analyzer, results are shown in FIG.1. After encapsulation, the particle size and surface potential of nanoparticles are changed, indicating that the chitosan-encapsulated nanoparticles have achieved surface potential reduction or reversal.

### Example 2

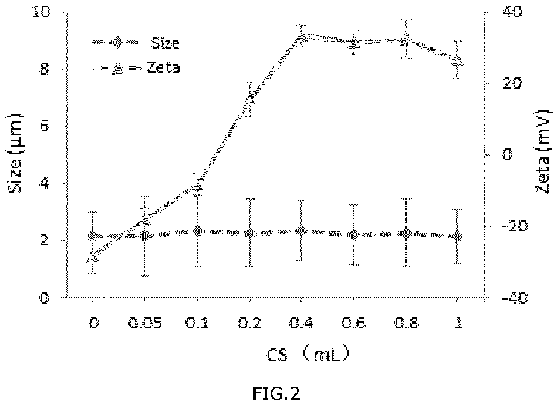

Seven tubes of 1 mL of polyacrylate nanoparticles are taken, added to 0.05,0.1,0.2,0.4,0.6,0.8,1.0 mL of 5% chitosan solution respectively, after mixed 3 hours, centrifuged to separate microbubbles, then the non-encapsulated chitosan is washed with pure water. The particle size and potential before and after crosslinking are measured by a particle counter and a particle size analyzer, results are shown in FIG.2. After encapsulation, the particle size and potential of microbubbles are changed, indicating that the chitosan-encapsulated nanoparticles have achieved surface potential reduction or reversal.

### Example 3

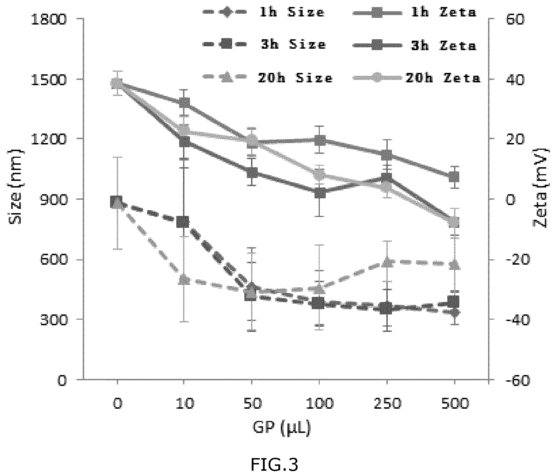

Five tubes of 1 mL of chitosan-encapsulated polyacrylate nanoparticles are taken and added to 10, 50, 100, 250, 500µL of 0.1 mol/L genipin solution respectively, after mixed 1 hour, 3 hours and 20 hours, centrifuged to separate the nano-particles, then the non-encapsulated genipin is washed with pure water. The particle size and potential before and after crosslinking are measured by a particle size analyzer, results are shown in FIG.3. After encapsulation, the particle size and surface potential of nanoparticles are changed, indicating that the genipin crosslinking can achieve the controlled adjustment of surface potential and particle size of nanoparticles.

### Example 4

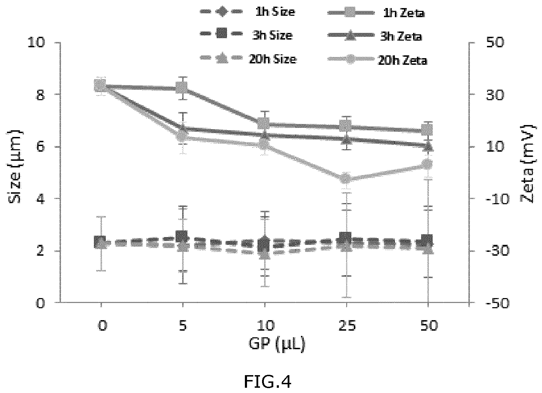

The chitosan-encapsulated (0.1 mL, 5wt%) polyacrylate microbubbles (4×1mL) in the Example 2 are taken, added to 5, 10, 25, 50 µL of 0.1 mol/L genipin solution respectively, after mixed 1 hour, 3 hours and 20 hours, centrifuged to separate the microbubbles, the non-encapsulated genipin is washed with pure water. The particle size and potential before and after crosslinking are measured by a particle counter and a particle size analyzer, results are shown in FIG.4. After encapsulation, the particle size and potential of microbubbles are changed, indicating that the genipin crosslinking can achieve the controlled adjustment of surface potential and particle size of nanoparticles.

### Example 5

Three tubes of 2 mL of chitosan-encapsulated (0.1 mL, 5wt%) polyacrylate microbubbles (4×1mL) are taken and mixed with 1 mg/L model drug adriamycin under magnetic stirring for 30 minutes, added to 0,40,400µL of 0.05 mol/L genipin solution (recorded as m-0, m-40 and m-400 respectively), after mixed 24 hours, centrifuged to separate the drug-loaded microbubbles, and the non-crosslinked genipin and non-encapsulated adriamycin are washed with pure water. The encapsulation efficiency and drug-loading rate of them are shown in FIG.5a. With the increase of genipin, the drug loading efficiency and encapsulation efficiency are increased significantly. The results of drug release are shown in FIG.5b, with the increase of crosslinking agent, the drug release rate is slowed down significantly.

All patents and publications mentioned in the description of the invention are the disclosed technologies in the art that can be used in the present invention. All patents and publications referenced herein are listed in the references, as separate reference of each publication. The invention described herein may be implemented in the absence of any element or elements, or with a limitation or a number of limitations, and such limitations are not specifically stated herein. For example, any one of the terms "including", "substantially consisting of..." and "consisting of...."used in each example can be substituted by any one of the other two terms. The terms and expressions used herein are descriptive rather than limitative. It is not intended to indicate that the terms and interpretations described herein have ruled out any equivalent features, but it should be understood that any changes or modifications can be made within the scope of the invention and claims herein. It should be aware that all embodiments herein are some preferred embodiments and features, and any person skilled in the art can make some changes and variations within the essence of the invention, and these changes and variations will be considered to fall into the scope of protection as restricted by the independent claims and dependent claims herein.

## Claims

1. A method for constructing micro-nanoparticles, the method comprising: using micro-nanoparticles as a substrate, adjusting the particle size and potential; using highly biocompatible material for surface encapsulation of the micro-nanoparticles, cross-linking modification on the micro-nanoparticles using the biocompatible cross-linking agent.

2. The method for constructing micro-nanoparticles according to claim 1, wherein the highly biocompatible material for surface encapsulation is chitosan.

3. The method for constructing micro-nanoparticles according to claim 1, wherein the biocompatible crosslinking agent is genipin.

4. The method for constructing micro-nanoparticles according to claim 1, wherein the method can adjust the particle diameter of the micro-nanoparticles.

5. The method for constructing micro-nanoparticles according to claim 1, wherein the method can adjust the surface potential of the micro-nano material.

6. The method for constructing micro-nanoparticles according to claim 1, wherein the method can improve the drug loading efficiency and rate of sustained release.

7. The method for constructing micro-nanoparticles according to claim 1, wherein the chitosan encapsulation modification method comprises the following steps:
a. Mix the aqueous solution of micro-nanoparticle with a 5% chitosan solution at 0.1∼1.0 for 1 to 3 hours to form a chitosan-encapsulated micro-nanoparticle;
b. Centrifuge to separate chitosan-encapsulated micro-nanoparticles, and wash to remove the non-encapsulated chitosan using pure water;
c. after concentration, redisperse the micro-nanoparticle in phosphate buffer (PPS, pH = 6.5-7.5);

8. The method for constructing micro-nanoparticles according to claim 7, wherein the genipin crosslinking modification method comprises the following steps:
a. Mix the chitosan-encapsulated micro-nanoparticle solution with 0.1 mol/L genipin solution at the volume ratio of 0.05∼0.5 for 1 to 24 hours;
b. Centrifuge to separate micro-nanoparticles, and wash to remove the unreacted genipin using pure water;
c. after concentration, redisperse the drug-loaded micro-nanoparticle in phosphate buffer(PPS, pH = 6.5-7.5).

9. The method for constructing micro-nanoparticles according to claim 8, wherein the genipin crosslinking loading method comprises the following steps:
a. Mix the chitosan-encapsulated micro-nanoparticle solution with appropriate amount of aqueous solution of a water-soluble drug, then add 0.1 mol/L genipin solution at the volume ratio of 0.05∼0.5 for 1 to 24 hours;
b. Centrifuge to separate micro-nanoparticles, and wash to remove the unreacted genipin and non-encapsulated drug using pure water;
c. after concentration, redisperse the drug-loaded micro-nanoparticle in phosphate buffer(PPS).

10. The method for constructing micro-nanoparticles according to claim 1, wherein the micro-nanoparticle is polyacrylate nanoparticle.

11. A biocompatible cross-linked micro-nano material, comprising a micro-nanoparticle, an externally encapsulated highly biocompatible surface encapsulation material chitosan, and an externally encapsulated genipin.

12. The biocompatible cross-linked micro-nano material according to claim 11, wherein the micro-nanoparticle is polyacrylate nanoparticle.

13. The biocompatible cross-linked micro-nano material, wherein the material is prepared according to the method as stated in any one of claims 1 to 10.
